(19) Europäisches Patentamt
European Patent Office
Office européen des brevets

(11) **EP 2 933 633 A1**

(12) **EUROPEAN PATENT APPLICATION**

(43) Date of publication:
**21.10.2015 Bulletin 2015/43**

(21) Application number: **15163580.2**

(22) Date of filing: **14.04.2015**

(51) Int Cl.:
**G01N 29/24** (2006.01)    **A61B 5/00** (2006.01)
**G01N 21/17** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **18.04.2014 JP 2014086369**

(71) Applicant: **Funai Electric Co., Ltd.
Daito-shi,
Osaka 574-0013 (JP)**

(72) Inventor: **Nakatsuka, Hitoshi
Daito-shi, Osaka 574-0013 (JP)**

(74) Representative: **Global IP Europe
Patentanwaltskanzlei
Pfarrstraße 14
80538 München (DE)**

(54) **Photoacoustic imaging device with triggered ultrasound detection**

(57)     A photoacoustic imaging device includes a light source that emits pulsed light at a subject, an ultrasonic transducer that converts vibration of a detection object of the subject that is generated according to the pulsed light to an electric signal, and a controller that selectively activates or deactivates the ultrasonic transducer, the controller deactivating the ultrasonic transducer while the light source emits the pulsed light.

FIG. 1

**Description**

CROSS-REFERENCE TO RELATED APPLICATIONS

This application claims priority to Japanese Patent Application No. 2014-086369 filed on April 18, 2014. The entire disclosure of Japanese Patent Application No. 2014-086369 is hereby incorporated herein by reference.

BACKGROUND

Field of the Invention

The present invention generally relates to a photoacoustic imaging device. More specifically, the present invention relates to a photoacoustic imaging device having an ultrasonic transducer and a light source that emits light at a subject.

Background Information

A photoacoustic imaging device having an ultrasonic transducer and a light source that emits light at a subject has been known in the art (see Japanese Laid-Open Patent Application Publication No. 2010-42158, for example).

Patent Literature 1 discloses an optical ultrasonic tomographic device equipped with a piezoelectric element and a light generating means for emitting pulsed light at a subject. This optical ultrasonic tomographic device is configured so that a pulse string having a plurality of aligned pulsed light beams is emitted as measurement light at a subject from a light generating means, and ultrasonic waves produced from the subject and originating in the emitted measurement light are sensed by a piezoelectric element disposed near the subject. This optical ultrasonic tomographic device also includes a tomographic image acquisition means, and this tomographic image acquisition means performs correlation processing of the sensed ultrasonic waves and the pulse string of measurement light, which increases the signal-to-noise (S/N) ratio in the sensed ultrasonic wave signal in imaging.

SUMMARY

When a light source is provided in close proximity to a subject in order to reduce the loss of light when light from a light generation means (light source) is emitted at (conducted to) a subject, the light source ends up being disposed extremely close to the piezoelectric element (ultrasonic transducer) that is disposed near the subject. In this case, when current for generating pulsed light is supplied to the light source, noise (electromagnetic waves and so forth) attributable to the current being supplied to the light source is generated near the ultrasonic transducer. Accordingly, this can lead to a problem in which the ultrasonic transducer is vibrated (mistakenly operated) by the noise. When noise thus causes a malfunction of the ultrasonic transducer, ultrasonic waves are generated from the ultrasonic transducer, and are reflected within the subject and detected by the ultrasonic transducer. Therefore, a conceivable problem is that a signal that has been affected by noise produced by the malfunctioning of the ultrasonic transducer will end up being acquired by the ultrasonic transducer and the reception circuit.

One aspect is to provide a photoacoustic imaging device with which the acquisition of signals originating in noise by the ultrasonic transducer and the reception circuit can be suppressed even when the light source is disposed near the subject.

Another aspect is to provide a photoacoustic imaging device that includes a light source configured to emit pulsed light at a subject, an ultrasonic transducer configured to convert vibration of a detection object of the subject that is generated according to the pulsed light to an electric signal, and a controller configured to selectively activate or deactivate the ultrasonic transducer, the controller deactivating the ultrasonic transducer while the light source emits the pulsed light.

In accordance with a preferred embodiment according to the photoacoustic imaging device mentioned above, the photoacoustic imaging device further comprises a reception circuit configured to acquire the electrical signal from the ultrasonic transducer.

In accordance with another preferred embodiment according to any one of the photoacoustic imaging devices mentioned above, the controller is configured to deactivate the ultrasonic transducer before the light source starts emitting the pulsed light, and activates the ultrasonic transducer after the light source stops emitting the pulsed light.

In accordance with another preferred embodiment according to any one of the photoacoustic imaging devices mentioned above, the controller is configured to activate the ultrasonic transducer before the vibration of the detection object of the subject is transmitted to the ultrasonic transducer.

In accordance with another preferred embodiment according to any one of the photoacoustic imaging devices mentioned above, the controller deactivates the ultrasonic transducer by setting potential at both ends of the ultrasonic transducer to be equal to each other.

In accordance with another preferred embodiment according to any one of the photoacoustic imaging devices mentioned above, the photoacoustic imaging device further comprises a switch electrically connected to the ultrasonic transducer, the controller is configured to set the potential at the both ends of the ultrasonic transducer to be equal to each other by the switch.

In accordance with another preferred embodiment according to any one of the photoacoustic imaging devices with the switch mentioned above, the photoacoustic imaging device further comprises a low-pass filter electrically connected between the controller and the switch.

In accordance with another preferred embodiment according to any one of the photoacoustic imaging devices with the switch mentioned above, the controller is configured to operate the switch with a control signal having a leading edge and a trailing edge.

In accordance with another preferred embodiment according to any one of the photoacoustic imaging devices with the switch mentioned above, the controller is configured to operate the switch such that the leading edge of the control signal includes a gradual leading edge and/or the trailing edge of the control signal includes a gradual trailing edge.

In accordance with another preferred embodiment according to any one of the photoacoustic imaging devices with the switch mentioned above, the controller is configured to operate the switch such that the switch deactivates the ultrasonic transducer in response to a level of the leading edge of the control signal reaching a predetermined deactivation level.

In accordance with another preferred embodiment according to any one of the photoacoustic imaging devices with the switch mentioned above, the controller is configured to operate the switch such that the switch activates the ultrasonic transducer in response to a level of the trailing edge of the control signal reaching a predetermined activation level.

In accordance with another preferred embodiment according to any one of the photoacoustic imaging devices with the switch mentioned above, the controller is configured to operate the switch such that a level of the leading edge of the control signal reaches a predetermined deactivation level to deactivate the ultrasonic transducer before the light source starts emitting the pulsed light.

In accordance with another preferred embodiment according to any one of the photoacoustic imaging devices mentioned above with the switch, the controller is configured to operate the switch such that a level of the trailing edge of the control signal reaching a predetermined activation level to activate the ultrasonic transducer after the light source stops emitting the pulsed light.

In accordance with another preferred embodiment according to any one of the photoacoustic imaging devices with the switch mentioned above, the photoacoustic imaging device further comprises a probe housing the light source, the ultrasonic transducer and the switch inside of the probe.

In accordance with another preferred embodiment according to any one of the photoacoustic imaging devices with the switch mentioned above, the photoacoustic imaging device further comprises a probe housing the light source and the ultrasonic transducer inside of the probe, and a device main body connected via a cable to the probe, with the controller and the switch being disposed inside of the device main body.

Also other objects, features, aspects and advantages of the present disclosure will become apparent to those skilled in the art from the following detailed description, which, taken in conjunction with the annexed drawings, discloses embodiments of the photoacoustic imaging device.

BRIEF DESCRIPTION OF THE DRAWINGS

Referring now to the attached drawings which form a part of this original disclosure:

FIG. 1 is a block diagram of the overall configuration of a photoacoustic imaging device in accordance with a first embodiment;
FIG. 2 is a side view of a probe of the photoacoustic imaging device in accordance with the first embodiment;
FIG. 3 is a diagram illustrating processing for deactivation of an ultrasonic transducer in the first embodiment;
FIG. 4 is a diagram illustrating an acoustic wave signal that has been affected by noise in a comparative photoacoustic imaging device;
FIG. 5 is a diagram obtained by imaging the acoustic wave signal that has been affected by noise in the comparative photoacoustic imaging device;
FIG. 6 is a diagram illustrating an acoustic wave signal in the first embodiment;
FIG. 7 is a diagram obtained by imaging the acoustic wave signal in the first embodiment;
FIG. 8 is a block diagram of the overall configuration of a photoacoustic imaging device in accordance with a second embodiment and a third embodiment;
FIG. 9 is a diagram illustrating processing for deactivation of an ultrasonic transducer in the third embodiment;
FIG. 10 is a diagram illustrating an acoustic wave signal that has been affected by noise originating in a switching component of a comparative photoacoustic imaging device;
FIG. 11 is a diagram illustrating an acoustic wave signal in the third embodiment;
FIG. 12 is a block diagram of the overall configuration of a photoacoustic imaging device in accordance with a modification example of the first embodiment;
FIG. 13 is a diagram illustrating processing for deactivation of an ultrasonic transducer in the modification example of the first embodiment; and
FIG. 14 is a diagram illustrating the configuration of a low-pass filter for an element deactivation signal.

DETAILED DESCRIPTION OF EMBODIMENTS

Selected embodiments will now be explained with reference to the drawings. It will be apparent to those skilled in the art from this disclosure that the following descriptions of the embodiments are provided for illustration only and not for the purpose of limiting the invention as defined by the appended claims and their equivalents.

FIRST EMBODIMENT

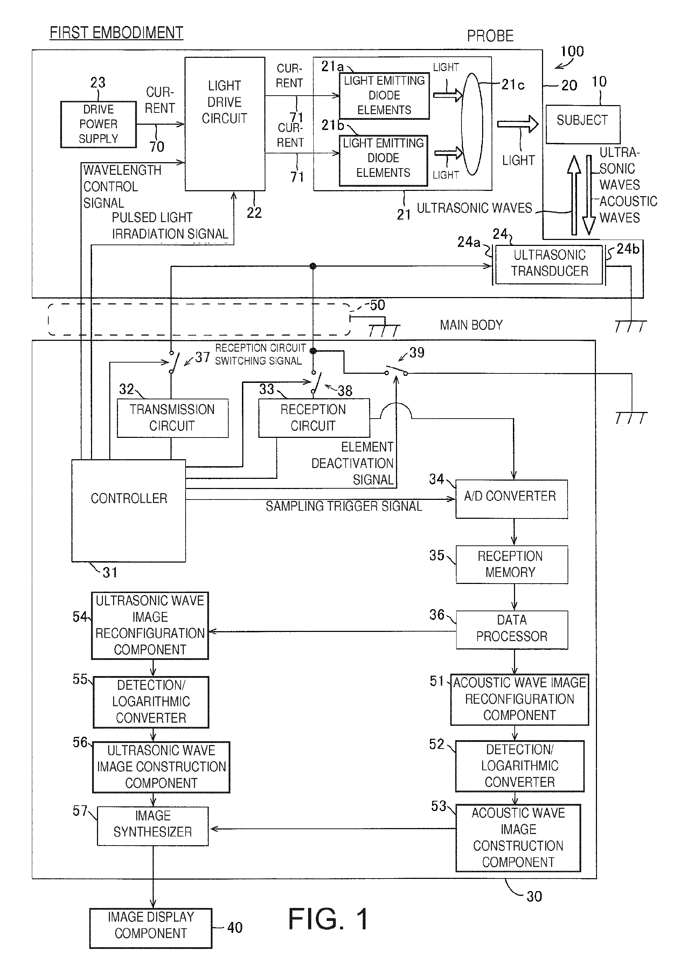

The configuration of a photoacoustic imaging device 100 in accordance with a first embodiment will be described through reference to FIGS. 1 to 3.

As shown in FIG. 1, the photoacoustic imaging device 100 in accordance with the first embodiment includes a probe 20 that detects acoustic wave signals from inside a subject 10, a main body 30 that processes and images the acoustic wave signals detected by the probe 20, and an image display component 40 that displays the images processed by the main body 30. The probe 20 and the main body 30 are connected via a cable 50 composed of a cable that is shielded against electromagnetic waves and is covered by a metal mesh, etc. The main body 30 is an example of the "device main body" of the present invention.

As shown in FIG. 1, the probe 20 includes a light source 21, a light drive circuit 22, a drive power supply 23, and an ultrasonic transducer 24. The light source 21 includes a plurality of light emitting diode elements 21 a (shown as a single block in FIG. 1) that generates pulsed light having a wavelength of approximately 760 nm, a plurality of light emitting diode elements 21 b (shown as a single block in FIG. 1) that generates pulsed light having a wavelength of approximately 850 nm, and a converging lens 21 c that converges the pulsed light from the light emitting diode elements 21 a and the light emitting diode elements 21 b and direct this pulsed light at the subject 10.

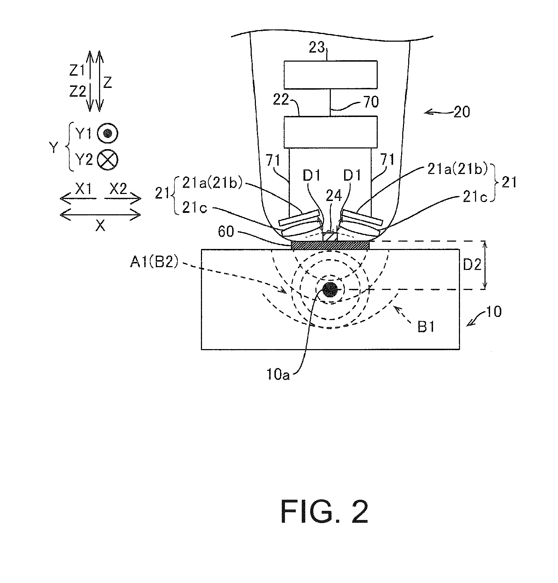

As shown in FIG. 2, the pulsed light emitted from the light source 21 at the subject 10 is absorbed by an optically absorbent substance in the subject 10 (the detection object 10a in FIG. 2 (such as hemoglobin)). The detection object 10a expands and contracts (returns from its expanded size to its original size) according to the intensity at which the pulsed light is emitted (the absorption amount), and this produces acoustic waves A1 from the detection object 10a. In this disclosure, for the sake of description, acoustic waves generated when the detection object 10a in the subject 10 absorbs light will be referred to separately as the "acoustic waves A1," while acoustic waves that are generated by the ultrasonic transducer 24 and reflected by the subject 10 will be referred to as "ultrasonic waves B2" (discussed below).

As shown in FIG. 1, the light drive circuit 22 is connected to the drive power supply 23 via an electrical wire 70, and power is supplied from the drive power supply 23. The drive power supply 23 acquires power from an external commercial power supply (not shown), and to convert this power into a voltage that is suited to the drive of the light emitting diode elements 21 a and 21 b. The light drive circuit 22 is connected to a controller 31 (discussed below). The light drive circuit 22 acquires a pulsed light irradiation signal and a wavelength control signal from the controller 31, and supplies power from the drive power supply 23 through electrical wires 71 to the light emitting diode elements 21 a and 21 b based on the acquired pulsed light irradiation signal and wavelength control signal. For example, the light drive circuit 22 supplies power with a peak current value of approximately 45 A and a pulse width of at least 100 ns and less than 200 ns to the light emitting diode elements 21 a and 21 b. Consequently, the light emitting diode elements 21 a and 21 b generates pulsed light with a waveform (pulse width) that is substantially the same as the waveform of the current supplied from the light drive circuit 22.

As shown in FIG. 1, the ultrasonic transducer 24 is made from lead zirconate titanate (PZT), for example, and includes a first end 24a (one end) and a second end 24b (the other end). The first end 24a of the ultrasonic transducer 24 is connected to a transmission switch 37, a reception switch 38, and a deactivation switch 39 via the cable 50, while the second end 24b of the ultrasonic transducer 24 is grounded.

The ultrasonic transducer 24 deforms according to the difference in potential between the first end 24a and the second end 24b (to expand when there is a potential difference). The ultrasonic transducer 24 here is configured so that a signal having a frequency of vibration (such as approximately 3 MHz) corresponding to the ultrasonic waves used for irradiating the subject 10 is issued from a transmission circuit 32 (discussed below) via the transmission switch 37. Consequently, the ultrasonic transducer 24 vibrates at a frequency of approximately 3 MHz and generates ultrasonic waves B1 (see FIG. 2).

As shown in FIG. 2, the ultrasonic waves B1 generated by the ultrasonic transducer 24 are reflected by a substance with high acoustic impedance inside the subject 10. For instance, let us assume that the detection object 10a in FIG. 2 has a high acoustic impedance. Let us also assume that the reflected ultrasonic waves are the ultrasonic waves B2. The reflected ultrasonic waves B2 then irradiate the ultrasonic transducer 24, and the ultrasonic transducer 24 vibrates.

The ultrasonic transducer 24 is configured so that if vibrations are produced by the ultrasonic waves B2 and the acoustic waves A1, a potential difference (acoustic wave signal) corresponding to the vibration will be produced between the first end 24a and the second end 24b. As shown in FIG. 1, the ultrasonic transducer 24 transmits acoustic wave signals through the reception switch 38 to a reception circuit 33.

Also, as shown in FIG. 1, the main body 30 is provided with the controller 31, the transmission circuit 32, the reception circuit 33, an A/D converter 34, a reception memory 35, a data processor 36, the transmission switch 37, the reception switch 38, the deactivation switch 39, an acoustic wave image reconfiguration component 51, a detection/logarithmic converter 52, an acoustic wave image construction component 53, an ultrasonic wave image reconfiguration component 54, detection/logarithmic converter 55, an ultrasonic wave image construction component 56, and an image synthesizer 57. The deactivation switch 39 is an example of the "switching component" of the present invention.

As shown in FIG. 1, the controller 31 includes a CPU (central processing unit) or the like, and controls the photoacoustic imaging device 100 by transmitting control signals to the various components. For instance, the controller 31 transmits the above-mentioned wavelength control signals and pulsed light irradiation signals to the light drive circuit 22. Also, the controller 31 controls the on-off switching (closing and opening) of the transmission switch 37, the reception switch 38, and the deactivation switch 39. The controller 31 transmits sampling trigger signals from the controller 31 to the reception memory 35 according to the pulsed light irradiation signals. The controller 31 can also include other conventional components such as an input interface circuit, an output interface circuit, and storage devices such as a ROM (Read Only Memory) device and a RAM (Random Access Memory) device. The controller 31 is programmed to control various components of the photoacoustic imaging device 100. The storage devices stores processing results and control programs. For example, the internal RAM of the controller stores statuses of operational flags and various control data. The internal ROM of the controller stores the programs for various operations. The controller 31 is capable of selectively controlling various components in accordance with the control program. It will be apparent to those skilled in the art from this disclosure that the precise structure and algorithms for controller 31 can be any combination of hardware and software that will carry out the functions of the present invention.

As discussed above, the transmission circuit 32 produces a potential difference between the first end 24a and the second end 24b of the ultrasonic transducer 24 based on a control signal from the controller 31, thereby producing the ultrasonic waves B1 from the ultrasonic transducer 24 (see FIG. 2).

The reception circuit 33 includes a coupling capacitor or the like, and acquires the AC component of voltage produced at the first end 24a of the ultrasonic transducer 24. The reception circuit 33 acquires the above-mentioned acoustic wave signal when the reception switch 38 is on and when the ultrasonic transducer 24 has produced vibration with the ultrasonic waves B2 and the acoustic waves A1 (see FIG. 2). The reception circuit 33 is connected to the A/D converter 34, and transmits the acquired acoustic wave signal to the A/D converter 34.

The A/D converter 34 converts an acoustic wave signal (analog signal) acquired from the reception circuit 33 into a digital signal according to the sampling trigger signal acquired from the controller 31. The A/D converter 34 is connected to the reception memory 35, and transmits an acoustic wave signal converted into a digital signal to the reception memory 35.

The reception memory 35 temporarily stores an acoustic wave signal converted into a digital signal. The reception memory 35 is connected to the data processor 36, and transmits the stored acoustic wave signals to the data processor 36.

The data processor 36 performs processing that separates the acoustic wave signals into signals of the acoustic waves A1 and signals of the ultrasonic waves B2. The data processor 36 is connected to the acoustic wave image reconfiguration component 51, and transmits data about the separated acoustic waves A1 to the acoustic wave image reconfiguration component 51.

The acoustic wave image reconfiguration component 51 performs processing to reconfigure data about the separated acoustic waves A1 as an image. The acoustic wave image reconfiguration component 51 is connected to the detection/logarithmic converter 52, and transmits data about the acoustic waves A1 reconfigured as an image to the detection/logarithmic converter 52.

The detection/logarithmic converter 52 performs waveform processing of the data reconfigured as an image. The detection/logarithmic converter 52 is connected to the acoustic wave image construction component 53, and transmits the data that has undergone waveform processing.

The acoustic wave image construction component 53 performs processing to construct a tomographic image of the inside of the subject 10 based on the data that has undergone waveform processing. The acoustic wave image construction component 53 is connected to the image synthesizer 57, and transmits a tomographic image based on the acoustic waves A1.

As shown in FIG. 1, the ultrasonic wave image reconfiguration component 54 of the main body 30 performs processing to reconfigure as an image the data about the ultrasonic waves B2 separated by the data processor 36. The ultrasonic wave image reconfiguration component 54 transmits a tomographic image based on the ultrasonic waves B2 through the detection/logarithmic converter 55 and the ultrasonic wave image construction component 56 to the image synthesizer 57.

The image synthesizer 57 performs processing to synthesize a tomographic image based on the acoustic waves A1 with a tomographic image based on the ultrasonic waves B2, and to output the synthesized image to the image display component 40.

The image display component 40 is constituted by a liquid crystal panel or the like, and displays an image inputted from the main body 30.

Utilizing the fact that the size relation of the absorption spectrum between oxidized hemoglobin and reduced hemoglobin inverts near a wavelength of approximately 800 nm, the data processor 36 and so on compute the difference in intensity between the acoustic waves A1 detected by the pulsed light of the light emitting diode elements 21 a and the acoustic waves A1 detected by the pulsed light of the light emitting diode elements 21 b, which makes it possible to detect whether more oxidized hemoglobin or more reduced hemoglobin is contained in blood. Consequently, arteries and veins can be distinguished from each other inside of the subject 10, and the result is displayed on the image display component 40.

Also, the transmission switch 37 of the main body 30 is provided between the transmission circuit 32 and the first end 24a of the ultrasonic transducer 24, and switches on and off (close and open) based on a control signal from the controller 31. When the transmission switch 37 is switched on, current can flow between the transmission circuit 32 and the first end 24a of the ultrasonic transducer 24.

The reception switch 38 of the main body 30 is provided between the reception circuit 33 and the deactivation switch 39 and the first end 24a of the ultrasonic transducer 24, and is configured to switch on and off (open and close) based on a reception circuit switching signal from the controller 31. When the reception switch 38 is switched on, current can flow between the reception circuit 33 and the deactivation switch 39 and the first end 24a of the ultrasonic transducer 24.

In this first embodiment, the photoacoustic imaging device 100 is configured such that the ultrasonic transducer 24 is deactivated by setting the potential to be substantially the same at the first end 24a and the second end 24b of the ultrasonic transducer 24. If there is no potential difference between the first end 24a and the second end 24b of the ultrasonic transducer 24 (if the potential is substantially the same at the first end 24a and the second end 24b), then the ultrasonic transducer 24 does not vibrate, and no ultrasonic waves B1 are produced. Here, "setting the potential to be substantially the same" or "setting the potential to be the same" means "setting the potential to be exactly the same" and/or "setting the potential such that the potential difference falls within a specific value range". Thus, if the ultrasonic transducer 24 does not vibrate while the potential difference between the first end 24a and the second end 24b of the ultrasonic transducer 24 falls within the specific value range, then the potentials of the first end 24a and the second end 24b does not need to be set to be exactly the same for deactivating the ultrasonic transducer 24. In this case, the ultrasonic transducer 24 can be deactivated by setting the potential difference between the first end 24a and the second end 24b of the ultrasonic transducer 24 to fall within the specific value range. This specific value range can be determined by the nature of the ultrasonic transducer 24.

More specifically, as shown in FIG. 1, the main body 30 includes the deactivation switch 39 that is connected to the ultrasonic transducer 24, and the controller 31 performs control to make the potential be substantially the same at the first end 24a and the second end 24b of the ultrasonic transducer 24 by controlling the deactivation switch 39. More precisely, the deactivation switch 39 is constituted by a field effect transistor (FET) or the like, and switches the deactivation switch 39 on and off (close and open) based on an element deactivation signal from the controller 31.

One side of the deactivation switch 39 is connected to the reception switch 38, the first end 24a of the ultrasonic transducer 24, and the transmission switch 37. The other side of the deactivation switch 39 is grounded. Consequently, when the deactivation switch 39 is switched on, the reception switch 38, the first end 24a of the ultrasonic transducer 24, and the transmission switch 37 are grounded. As a result, since the second end 24b of the ultrasonic transducer 24 is also grounded, the first end 24a and the second end 24b of the ultrasonic transducer 24 have substantially the same potential.

As shown in FIG. 2, with the probe 20, the ultrasonic transducer 24 is disposed near the end face on the subject 10 side (the Z2 direction side) of the probe 20, so as to be close to the subject 10. Also, the light emitting diode elements 21 a and 21 b and the converging lens 21 c are similar to the ultrasonic transducer 24 in that they are disposed on the subject 10 side (the Z2 direction side) of the probe 20, so as to be close to the subject 10. That is, the ultrasonic transducer 24 and the light emitting diode elements 21 a and 21 b are provided so as to be close together (separated by a distance of D1).

The probe 20 includes one pair of sets each having the light emitting diode element 21 a, the light emitting diode element 21 b and the converging lens 21 c, and the ultrasonic transducer 24 is disposed between the pair of the sets so as to be sandwiched therebetween. The light drive circuit 22 and the drive power supply 23 in the probe 20 are disposed more on the opposite side from the subject 10 side (the Z1 direction side) than the light source 21 and the ultrasonic transducer 24.

The probe 20 detects acoustic waves and ultrasonic waves in a state in which a transparent gel layer 60 that transmits light is interposed between the face on the subject 10 side (the Z2 direction side) of the probe 20 in which the ultrasonic transducer 24 is disposed, and the subject 10. This gel layer 60 has a refractive index that is substantially the same as that of the surface of the subject 10, and suppresses reflection of the light emitted from the light source 21 at the subject 10, by the surface of the subject 10. The gel layer 60 also functions as a propagation substance for efficiently propagating the acoustic waves A1 generated from the detection object 10a of the subject 10, and the ultrasonic waves B2 reflected from the detection object 10a, to the ultrasonic transducer 24.

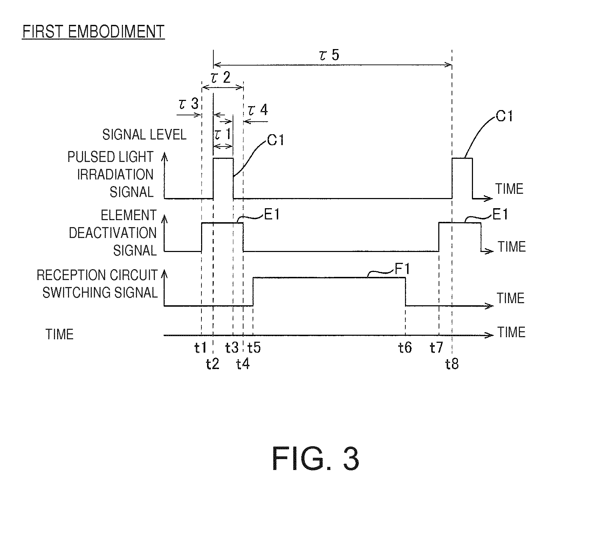

As shown in FIG. 3, in the first embodiment, the controller 31 performs control to deactivate the ultrasonic transducer 24 for a specific time period $\tau 2$ that includes the time period $\tau 1$ in which pulsed light is generated by the light emitting diode elements 21 a and 21 b. The specific time period $\tau 2$ includes the time period $\tau 3$ before pulsed light is generated by the light emitting diode elements 21 a and 21 b, and the time period $\tau 4$ after the pulsed light is generated, in addition to the time period $\tau 1$ in which the pulsed light is generated by the light emitting diode elements 21 a and 21 b. That is, the time pe-

riods $\tau1$ to $\tau4$ have the relation expressed by the following Formula (1). Here, the time periods $\tau1$ to $\tau4$ can be positive (larger than zero), for example.

$$\tau2 = \tau1 + \tau3 + \tau4 \ldots (1)$$

As shown in FIG. 3, the controller 31 transmits pulsed light irradiation signals C1 each having the time period $\tau1$ (from a time t2 until a time t3) to the light drive circuit 22 at a time interval $\tau5$ (the time interval from the time t2 until a time t8). The controller 31 transmits element deactivation signals E1 each having the time period $\tau2$ (from a time t1 until a time t4) to the deactivation switch 39 at the time interval $\tau5$ (the time interval from the time t1 until a time t7).

As shown in FIG. 3, in the first embodiment, the specific time period $\tau2$ is made up of the time period up to when the reception circuit 33 starts to acquire the acoustic wave signal. More specifically, the controller 31 transmits a reception circuit switching signal F1 to the reception switch 38 from the time t5 (a time that is later than the time t4) to the time t6 (a time that is before the time t7). Specifically, the reception circuit 33 starts acquiring the acoustic wave signal at the time t5 after the time t4, and the specific time period $\tau2$ (from the time t1 to the time t4) includes a time period prior up to the time t5.

As shown in FIG. 3, the length of the time period $\tau4$ after the pulsed light is generated is less than the length of the time period $\tau1$ in which the pulsed light is generated. That is, the time period $\tau1$ and the time period $\tau4$ have the relation expressed by the following Formula (2).

$$\tau4 \leq \tau1 \ldots (2)$$

The time period $\tau1$ in which the pulsed light is generated corresponds to the resolution for generating the acoustic waves A1, so if the length of the time period $\tau4$ after the pulsed light is generated is kept to time period $\tau1$ or less, it will be less likely that the acquisition period of the acoustic wave signal acquired by the reception circuit 33 will be too short. For example, if the length of the time period $\tau1$ in which the pulsed light is generated is 100 ns, and the speed of sound within the subject 10 is 1500 m/s, then the resolution will be 0.15 mm. If the length of the time period $\tau4$ after the pulsed light is generated is less than 100 ns, the reception circuit 33 will be able to acquire the acoustic wave signal from the ultrasonic transducer 24 after the subject 10 has been irradiated with the pulsed light (100 ns later).

In this case, if the thickness of the gel layer 60 is approximately 0.15 mm, then the detection object 10a can be detected even if it is near the surface inside the subject 10. If the thickness of the gel layer 60 is over 0.15 mm, or if the main body 30 does not image the area near the surface inside the subject 10, then the time period $\tau4$ or the start of acquisition of the acoustic wave signal by the reception circuit 33 (the time t5) can be adjusted according to the thickness of the gel layer 60 or to the thickness of the surface inside the subject 10 that is not imaged.

The imaging processing of an acoustic wave signal that has been affected by noise, when using a comparative photoacoustic imaging device in which the ultrasonic transducer is not deactivated, in a time period in which the light source generates pulsed light will now be described through reference to FIGS. 2, 4, and 5.

With the comparative photoacoustic imaging device, when the probe is configured as in FIG. 2, the acoustic wave signal will sometimes be affected by noise. More specifically, for the light source to generate pulsed light, a current with a peak value of approximately 45 A and a pulse width of at least 100 ns and less than 200 nm is allowed to flow from the light drive circuit to the light source. In this case, electromagnetic induction attributable to the flow of current occurs and radiation noise (electromagnetic waves) and the like are generated near the light source. Since the light source and the ultrasonic transducer are disposed close together, a potential difference attributable to radiation noise or the like occurs at the two ends of the ultrasonic transducer, and the ultrasonic transducer vibrates (malfunctions).

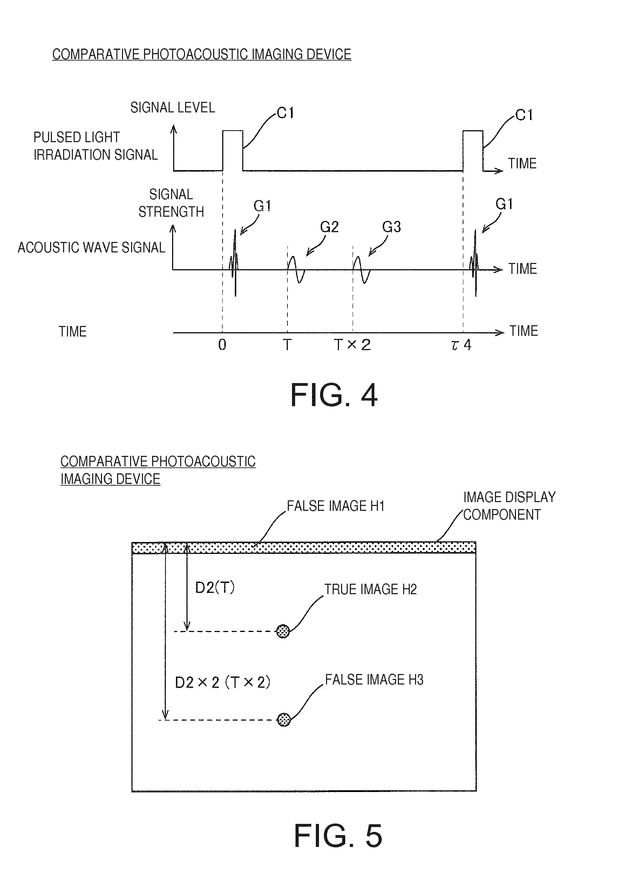

The reception circuit then acquires the potential difference at the ends of the ultrasonic transducer that occurred because of radiation noise or the like. Specifically, as shown in FIG. 4, the reception circuit acquires an acoustic wave signal G1 from the ultrasonic transducer. As shown in FIG. 5, a false image H1 corresponding to the acoustic wave signal G1 is displayed on the image display component.

As shown in FIG. 2, pulsed light from the light source proceeds through the subject at a speed of approximately $3.0 \times 10^8$ m/s, and then reaches the detection object (the light source and the detection object have a distance D2). The detection object then absorbs the pulsed light and emits an acoustic wave. The acoustic wave emitted from the detection object proceeds through the subject at a speed of approximately 1500 m/s, and reaches the ultrasonic transducer after a time T has elapsed since the acoustic wave was generated. In this case, the speed at which the pulsed light proceeds through the subject is sufficiently higher than the speed at which the acoustic wave proceeds through the subject, so the time during which the pulsed light proceeds through the subject can be ignored. Consequently, the time period until the ultrasonic transducer is vibrated by the acoustic wave after the pulsed light is emitted from the light source is approximately the time T. That is, as shown in FIG. 4, the reception circuit acquires an acoustic wave signal G2 from the ultrasonic transducer. Then, as shown in FIG. 5, a true image H2 corresponding to the acoustic wave signal G2 is displayed on the image display component.

As shown in FIG. 2, the inside of the subject is irradiated with ultrasonic waves produced by the malfunctioning of the ultrasonic transducer. The ultrasonic waves produced by this malfunctioning proceed through the subject at a speed of approximately 1500 m/s, and reach the detection object after the time T since the ultrasonic waves were generated by the malfunction. The ultrasonic waves produced by malfunction are then reflected by the detection object, and reach the ultrasonic transducer after another time T. The ultrasonic transducer then vibrates after approximately time T x 2 (twice the length of T) since the malfunction. Specifically, as shown in FIG. 4, the reception circuit acquires an acoustic wave signal G3 from the ultrasonic transducer. Then, as shown in FIG. 5, a false image H3 corresponding to the acoustic wave signal G3 is displayed on the image display component. As discussed above, when the probe is configured as in FIG. 2 in a comparative photoacoustic imaging device, the unnecessary false images H1 and H3 are displayed on the image display component along with the true image H2.

The imaging processing of an acoustic wave signal in the photoacoustic imaging device 100 in accordance with the first embodiment will now be described through reference to FIGS. 2, 6, and 7.

With the photoacoustic imaging device 100 in accordance with the first embodiment, a current with a peak value of approximately 45 A and a pulse width of at least 100 ns and less than 200 ns is allowed to flow from the light drive circuit 22 to the light emitting diode elements 21 a and 21 b in order for pulsed light to be generated by the light emitting diode elements 21 a and 21 b. In this case, electromagnetic induction attributable to the flow of current occurs and radiation noise (electromagnetic waves) and the like are generated near the light emitting diode elements 21 a and 21 b. On the other hand, while pulsed light is being generated by the light emitting diode elements 21 a and 21 b (C1 in FIG. 3), the deactivation switch 39 is switched on, the potential at the first end 24a is substantially the same as that at the second end 24b, and the ultrasonic transducer 24 is deactivated. This makes it less likely that the ultrasonic transducer 24 will vibrate (malfunction) due to radiation noise or the like.

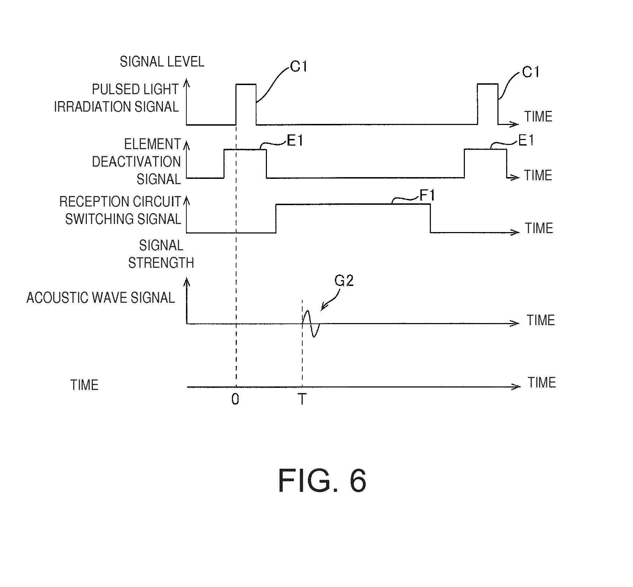

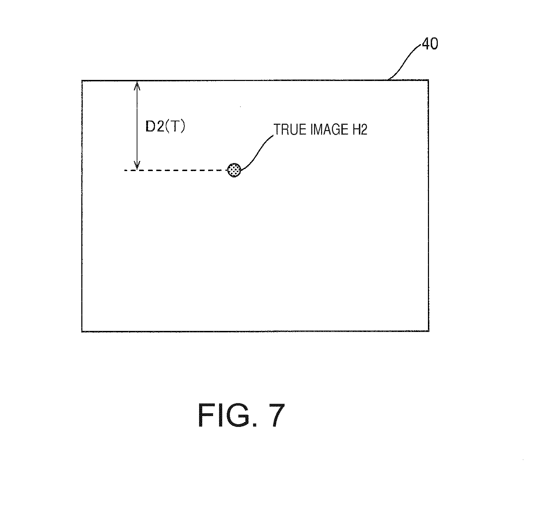

As shown in FIG. 6, the reception circuit 33 acquires the acoustic waves A1 emitted from the detection object 10a in the subject 10 by having the light source 21 irradiate the inside of the subject 10 with pulsed light. Specifically, the reception circuit 33 acquires the acoustic wave signal G2. Because the ultrasonic transducer 24 does not malfunction, the acoustic wave signals G1 and G3 in FIG. 4 are not acquired by the reception circuit 33. As a result, as shown in FIG. 7, only the true image H2 is displayed on the image display component 40, and the false images H1 and H3 are not displayed, unlike when the comparative photoacoustic imaging device is used (see FIG. 5).

The following effects are obtained with the first embodiment.

With the first embodiment, as discussed above, the controller 31 is configured to perform control to deactivate the ultrasonic transducer 24 during the specific time period τ2 (see FIG. 3) that overlaps the time period τ1 in which the light emitting diode elements 21 a and 21 b generate pulsed light. Consequently, since the ultrasonic transducer 24 is deactivated during the specific time period τ2 that overlaps the time period in which noise (electromagnetic waves and the like) is produced because of the flow of current for generating pulsed light at the light emitting diode elements 21 a and 21 b (a time period substantially equal to the time period τ1), it is less likely that the ultrasonic transducer 24 will malfunction (vibrate). As a result, it is less likely that the reception circuit 33 and the ultrasonic transducer 24 will acquire a signal that has been affected by noise, even when the light source 21 is disposed near the subject 10.

With the first embodiment, as discussed above, the specific time period τ2 is configured to include at least the time period τ1 in which the light emitting diode elements 21 a and 21 b generate pulsed light. Consequently, the ultrasonic transducer 24 is deactivated during the time period τ1 in which pulsed light is being generated, so it is less likely that the ultrasonic transducer 24 will malfunction (vibrate).

With the first embodiment, as discussed above, the specific time period τ2 is configured to include the time period τ3 prior to the time period τ1 in which the light emitting diode elements 21 a and 21 b generate pulsed light and the time period τ4 that comes after this time period τ1, in addition to the time period τ1 in which the light emitting diode elements 21 a and 21 b generate pulsed light. Consequently, the ultrasonic transducer 24 is deactivated not only during the time period τ1 in which the light emitting diode elements 21 a and 21 b generate pulsed light, but also during the time period τ3 and the time period τ4 that come before and after the time period τ1 in which the light emitting diode elements 21 a and 21 b generate pulsed light, so it is even less likely that the ultrasonic transducer 24 will malfunction (vibrate).

With the first embodiment, as discussed above, the controller 31 is configured to perform control so that the reception circuit 33 starts acquiring an acoustic wave signal after the time period τ1 in which the light emitting diode elements 21 a and 21 b generate pulsed light, and the specific time period τ2 is configured to include the time period τ1 in which the light emitting diode elements 21 a and 21 b generate pulsed light and also includes the time period prior to when the reception circuit 33 starts acquiring the acoustic wave signal (a time period prior to the time t5 in FIG. 3). Consequently, the specific time period τ2 ends (time t4) by the time the reception circuit 33 starts acquiring the acoustic wave signal (time t5), so it is less likely that providing the specific time period τ2 in which the ultrasonic transducer 24 is deactivated will reduce the time period in which the reception circuit 33 acquires the acoustic wave signal (a time period from the time t5 until the time t6).

With the first embodiment, as discussed above, the controller 31 is configured to perform control to deactivate the ultrasonic transducer 24 by having the potential be substantially the same at the two ends of the ultrasonic transducer 24 (the first end 24a and the second end 24b) during the specific time period τ2. Consequently, since there is no difference in potential between the two ends of the ultrasonic transducer 24 (the first end 24a and the second end 24b), the ultrasonic transducer 24 can be easily deactivated.

With the first embodiment, as discussed above, there is further provided the deactivation switch 39 that is connected to the ultrasonic transducer 24, and the controller 31 is configured to perform control to make the potential substantially the same at both ends of the ultrasonic transducer 24 (the first end 24a and the second end 24b) by controlling the deactivation switch 39. Consequently, the potential can be easily made the same at both ends of the ultrasonic transducer 24 (the first end 24a and the second end 24b) by controlling the deactivation switch 39.

With the first embodiment, as discussed above, the photoacoustic imaging device 100 comprises the probe 20 in the interior of which are disposed the light source 21 and the ultrasonic transducer 24, and which is configured to be able to irradiate the subject 10 with pulsed light from the light source 21 by being disposed close to the subject 10, and the main body 30 that is connected via the cable 50 to the probe 20 and in the interior of which are disposed the reception circuit 33 and the controller 31. The deactivation switch 39 is disposed in the interior of the main body 30. Consequently, unlike when the deactivation switch 39 is disposed at the probe 20, there is no need for wiring to the cable 50 to control the deactivation switch 39. As a result, the configuration of the photoacoustic imaging device 100 can be simplified to an extent corresponding to the fact that no wiring to the cable 50 is needed to control the deactivation switch 39.

With the first embodiment, as discussed above, the light source 21 is configured to include the light emitting diode elements 21 a and 21 b that are capable of generating pulsed light. Consequently, unlike when using a light emitting element that emits a laser beam, there is no need for the optical members to be precisely aligned (positioned), nor are an optical bench and a sturdy housing required for suppressing fluctuation of the characteristics due to vibration of the optical system. As a result, since there is no need for precise alignment of optical members, and no need for an optical bench or a sturdy housing, the size and complexity of the photoacoustic imaging device 100 can be correspondingly reduced.

In the illustrated embodiment, the photoacoustic imaging device 100 comprises the light source 21 that emits pulsed light at the subject 10, the ultrasonic transducer 24 that converts the vibration of the detection object 10a of the subject 10 that is generated according to the pulsed light to the electric signal, and the controller 31 that selectively activates or deactivates the ultrasonic transducer 24, the controller 31 deactivating the ultrasonic transducer 24 while the light source 21 emits the pulsed light.

The photoacoustic imaging device 100 can further comprise the reception circuit 33 that acquires the electrical signal from the ultrasonic transducer 24.

Also, with the photoacoustic imaging device 100, the controller 31 can deactivate the ultrasonic transducer 24 before the light source 21 starts emitting the pulsed light, and activate the ultrasonic transducer 24 after the light source 21 stops emitting the pulsed light.

With the photoacoustic imaging device 100, the controller 31 can activate the ultrasonic transducer 24 before the vibration of the detection object 10a of the subject 10 is transmitted to the ultrasonic transducer 24.

With the photoacoustic imaging device 100, the controller 31 can deactivate the ultrasonic transducer 24 by setting potential at both ends 24a and 24b of the ultrasonic transducer 24 to be equal to each other.

The photoacoustic imaging device 100 can further comprise the deactivation switch 39 electrically connected to the ultrasonic transducer 24. The controller 31 can set the potential at the both ends 24a and 24b of the ultrasonic transducer 24 to be equal to each other by the deactivation switch 39.

With the photoacoustic imaging device 100, the controller 31 can operate the deactivation switch 39 with the element deactivation signal E1 (e.g., control signal) having the leading edge (at time t1) and the trailing edge (at time t4).

With the photoacoustic imaging device 100, the deactivation switch 39 can deactivate the ultrasonic transducer 24 in response to a level of the leading edge of the element deactivation signal E1 (e.g., the control signal) reaching a predetermined deactivation level. For example, as illustrated in FIG. 3, when the element deactivation signal E1 has a rectangular wave, the predetermined deactivation level can be equal to or less than the amplitude (maximum amplitude) of the rectangular wave.

With the photoacoustic imaging device 100, the deactivation switch 39 can activate the ultrasonic transducer 24 in response to a level of the trailing edge of the element deactivation signal E1 (e.g., the control signal) reaching a predetermined activation level. For example, as illustrated in FIG. 3, when the element deactivation signal E1 has a rectangular wave, the predetermined activation level can be equal to or less than the amplitude (maximum amplitude) of the rectangular wave.

With the photoacoustic imaging device 100, a level of the leading edge of the element deactivation signal E1 (e.g., the control signal) reaches a predetermined deactivation level (at time t1) to deactivate the ultrasonic transducer 24 before the light source 21 starts emitting the pulsed light (at time t2). For example, as illustrated in FIG. 3, when the element deactivation signal E1 has a rectangular wave, the predetermined deactivation level can be equal to or less than the amplitude (maximum amplitude) of the rectangular wave.

With the photoacoustic imaging device 100, a level of the trailing edge of the element deactivation signal E1 (e.g., the control signal) reaches a predetermined activation level (at time t4) to activate the ultrasonic transducer 24 after the light source 21 stops emitting the pulsed light (at time t3). For example, as illustrated in FIG. 3, when the element deactivation signal E1 has a rectangular wave, the predetermined activation level can be equal to or less than the amplitude (maximum amplitude) of the rectangular wave.

The photoacoustic imaging device 100 can further comprise the probe 20 housing the light source 21 and the ultrasonic transducer 24 inside of the probe 20, and the main body 30 (e.g., the device main body) connected via the cable 50 to the probe 20, with the controller 31 and the deactivation switch 39 being disposed inside of the main body 30 (e.g., the device main body).

With the photoacoustic imaging device 100 the light source 21 can include the light emitting diode elements 21 a and 21 b that emits the pulsed light.

SECOND EMBODIMENT

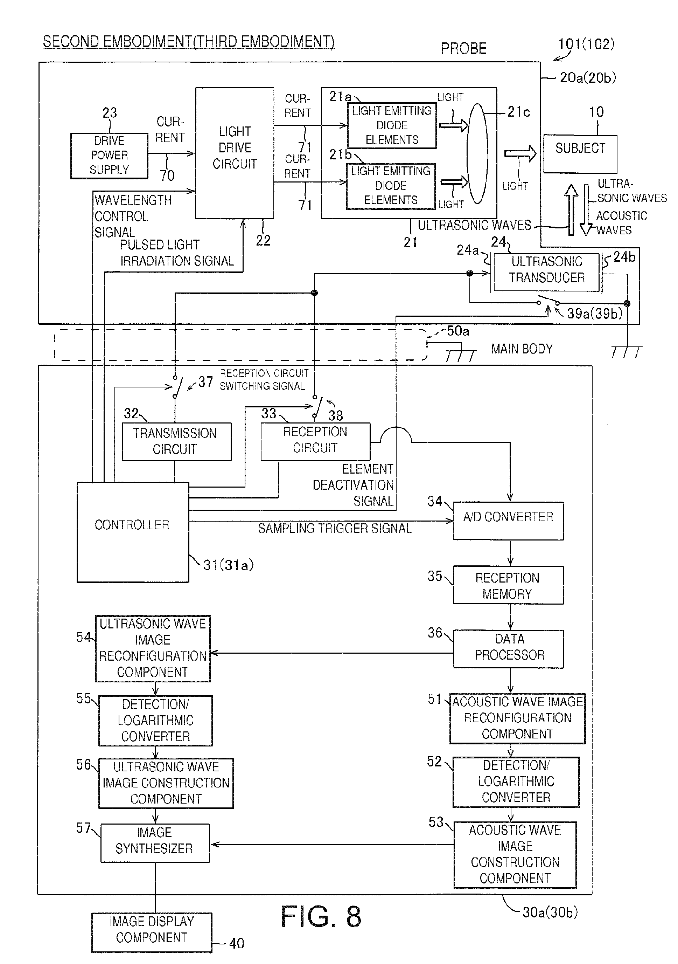

The configuration of a photoacoustic imaging device 101 in accordance with a second embodiment will now be described through reference to FIG. 8. In view of the similarity between the first and second embodiments, the parts of the second embodiment that are identical to the parts of the first embodiment will be given the same reference numerals as the parts of the first embodiment. Moreover, the descriptions of the parts of the second embodiment that are identical to the parts of the first embodiment may be omitted for the sake of brevity. In the second embodiment, unlike with the photoacoustic imaging device 100 in which the deactivation switch 39 is provided to the main body 30, a deactivation switch 39a is provided to a probe 20a.

As shown in FIG. 8, the photoacoustic imaging device 101 in accordance with the second embodiment includes the probe 20a that irradiates the subject 10 with pulsed light from the light source 21 by disposing the light source 21 and the ultrasonic transducer 24 in the interior, and by disposing them near the subject 10, and a main body 30a that is connected to the probe 20a via a cable 50a. The deactivation switch 39a is disposed in the interior of the probe 20a. The cable 50a includes wiring for connecting the controller 31 and the deactivation switch 39a, in addition to the wiring included in the interior of the cable 50 as in the first embodiment. The components included in the main body 30a are the same as in the main body 30 in the first embodiment, except that the deactivation switch 39 is excluded from the interior.

As shown in FIG. 8, one side of the deactivation switch 39a is connected to the first end 24a of the ultrasonic transducer 24, and the other side of the deactivation switch 39a is connected to the second end 24b of the ultrasonic transducer 24. Consequently, when the deactivation switch 39a is switched on (closed) by acquiring an element deactivation signal from the controller 31, the first end 24a and the second end 24b of the ultrasonic transducer 24 form a short circuit. Because of this short-circuiting of the first end 24a and the second end 24b of the ultrasonic transducer 24, the potential is substantially the same at the first end 24a and the second end 24b of the ultrasonic transducer 24. Also, since the other side of the deactivation switch 39a is grounded, when the deactivation switch 39a is switched on, the potential of the first end 24a and the second end 24b of the ultrasonic transducer 24 is grounded.

The deactivation switch 39a and the ultrasonic transducer 24 are both disposed near the interior of the probe 20a. Consequently, compared to when the deactivation switch 39a is provided to the main body 30a and the ultrasonic transducer 24 is provided to the probe 20a, the wiring is shorter between the deactivation switch 39a and the ultrasonic transducer 24. As a result, the impedance (resistance, floating capacity, etc.) between the deactivation switch 39a and the ultrasonic transducer 24 is reduced by an amount proportional to the reduction in length of the wiring between the deactivation switch 39a and the ultrasonic transducer 24. If the wiring impedance is high, it will sometimes be difficult to make the potential substantially the same at both ends of the ultrasonic transducer 24.

As shown in FIG. 8, the processing of the controller 31 for deactivating the ultrasonic transducer 24 in the second embodiment is the same as the processing of the controller 31 in the first embodiment (see FIGS. 3, 6, and 7). Specifically, the ultrasonic transducer 24 in the second embodiment is similar to the ultrasonic transducer 24 in the first embodiment in that the effect of noise (electromagnetic waves and so forth) produced by the flow of current near the ultrasonic transducer 24 is suppressed by making the potential substantially the same at both ends of the ultrasonic transducer 24 during the time period in which pulsed light is generated by the light emitting diode elements 21 a and 21 b. The rest of the configuration of the photoacoustic imaging device 101 in the second embodiment is the same as that of the photoacoustic imaging device 100 in the first embodiment.

The following effects are obtained with the second embodiment.

With the second embodiment, as discussed above, the light source 21 and the ultrasonic transducer 24 are disposed in the interior of the photoacoustic imaging device 101, and are disposed near the subject 10, which allows the probe 20a to be able to irradiate the subject 10 with pulsed light from the light source 21. The deactivation switch 39a is disposed in the interior of the probe 20a. Since the ultrasonic transducer 24 and the deactivation switch 39a are thus disposed close together, impedance (resistance, floating capacity, etc.) between the ultrasonic transducer 24 and the deactivation switch 39a can be lower than when the ultrasonic transducer 24 and the deactivation switch 39a are disposed farther apart. As a result, the potential at both ends of the ultrasonic transducer 24 can more reliably be made substantially the same. The rest of the effects of the photoacoustic imaging device 101 in the second embodiment are the same as those of the photoacoustic imaging device 100 in the first embodiment.

In the illustrated embodiment, the photoacoustic imaging device 101 comprises the light source 21 that emits pulsed light at the subject 10, the ultrasonic transducer 24 that converts the vibration of the detection object 10a of the subject 10 that is generated according to the pulsed light to the electric signal, and the controller 31 that selectively activates or deactivates the ultrasonic transducer 24, the controller 31 deactivating the ultrasonic transducer 24 while the light source 21 emits the pulsed light.

The photoacoustic imaging device 101 can further comprise the reception circuit 33 that acquires the electrical signal from the ultrasonic transducer 24.

Also, with the photoacoustic imaging device 101, the controller 31 can deactivate the ultrasonic transducer 24 before the light source 21 starts emitting the pulsed light, and activate the ultrasonic transducer 24 after the light source 21 stops emitting the pulsed light.

With the photoacoustic imaging device 101, the controller 31 can activate the ultrasonic transducer 24 before the vibration of the detection object 10a of the subject 10 is transmitted to the ultrasonic transducer 24.

With the photoacoustic imaging device 101, the controller 31 can deactivate the ultrasonic transducer 24 by setting potential at both ends 24a and 24b of the ultrasonic transducer 24 to be equal to each other.

The photoacoustic imaging device 101 can further comprise the deactivation switch 39a electrically connected to the ultrasonic transducer 24. The controller 31 can set the potential at the both ends 24a and 24b of the ultrasonic transducer 24 to be equal to each other by the deactivation switch 39a.

The photoacoustic imaging device 101 can further comprise the probe 20a housing the light source 21, the ultrasonic transducer 24 and the deactivation switch 39a inside of the probe 20a.

With the photoacoustic imaging device 101, the light source 21 can include the light emitting diode elements 21 a and 21 b that emits the pulsed light.

THIRD EMBODIMENT

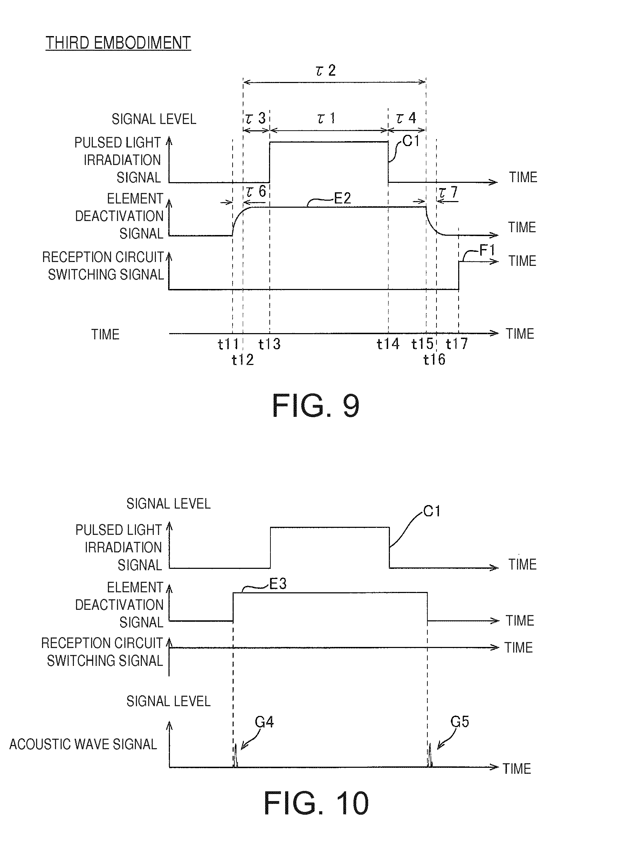

The configuration of a photoacoustic imaging device 102 in accordance with a third embodiment will now be described through reference to FIGS. 8 and 9. In view of the similarity between the first to third embodiments, the parts of the third embodiment that are identical to the parts of the first or second embodiment will be given the same reference numerals as the parts of the first or second embodiment. Moreover, the descriptions of the parts of the third embodiment that are identical to the parts of the first or second embodiment may be omitted for the sake of brevity. In the third embodiment, a controller 31 a is configured so that the leading edge and trailing edge of the waveform of the element deactivation signal for controlling the deactivation switching will have a shape that is blunter or more gradual than a rectangular waveform.

As shown in FIG. 8, the photoacoustic imaging device 102 in the third embodiment is similar to the photoacoustic imaging device 101 in the second embodiment in that it includes a probe 20b that irradiates the subject 10 with pulsed light from the light source 21 by disposing the light source 21 and the ultrasonic transducer 24 in the interior and disposing them near the subject 10, and a main body 30b that is connected via a cable 50a to the probe 20b. A deactivation switch 39b is disposed in the interior of the probe 20b. The main body 30b includes a controller 31 a. The deactivation switch 39b is similar to the deactivation switch 39 in the first embodiment in that it is constituted by a field effect transistor (FET) or the like, and controls the amount of current flowing between the two ends (between the drain and source) of the deactivation switch 39b according to the level of the element deactivation signal from the controller 31 a (the voltage between the gate and the source).

In the third embodiment, the controller 31 a is configured so that the leading edge and trailing edge of a waveform E2 of the element deactivation signal for controlling the deactivation switch 39b have a shape that is blunter or more gradual than a rectangular waveform. More specifically, as shown in FIG. 9, the controller 31 a performs control to gradually lift the leading edge of the waveform E2 of the element deactivation signal (the time period from a time t11 to a time t12) up to a signal level that goes from off to on at a time constant $\tau6$. The controller 31 a perform control to gradually lower the trailing edge of the waveform E2 of the element deactivation signal (between a time t15 and a time t16) to a signal level that goes from on to off at a time constant $\tau7$. The time constants $\tau6$ and $\tau7$ can be equivalent.

As shown in FIG. 9, the controller 31 a is similar to the controller 31 in the first embodiment in that it performs control to deactivate the ultrasonic transducer 24 in the specific time period $\tau2$ that includes the time period $\tau1$ in which the light emitting diode elements 21 a and 21 b generate pulsed light (a time period from the time t12 to the time t15). The specific time period $\tau2$ includes not only the time period $\tau1$ in which the light emitting diode elements 21 a and 21 b generate pulsed light, but also a time period $\tau3$ prior to when the light emitting diode elements 21 a and 21 b generate pulsed light, and a time period $\tau4$ after the pulsed light is generated.

As shown in FIG. 9, the specific time period $\tau2$ is similar to the specific time period $\tau2$ in the first embodiment in that it is made up of a time period before the reception circuit 33 starts acquiring the acoustic wave signal. More specifically, the controller 31 a starts the acquisition of the acoustic wave signal by the reception circuit 33 at a time t17 that is later than the time t16. The rest of the configuration of the photoacoustic imaging device 102 in the third embodiment is the same as that of the photoacoustic imaging device 100 in the first embodiment.

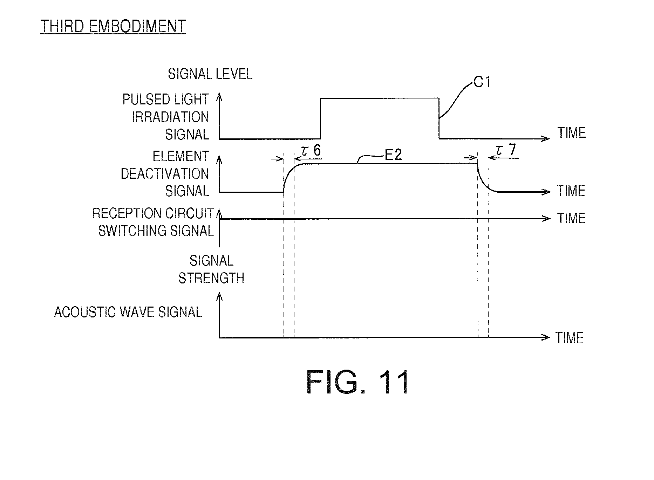

The principle behind suppressing noise generation attributable to deactivation switching, by using the waveform E2 of the element deactivation signal having a blunt or gradual shape in the photoacoustic imaging device 102 of the third embodiment will now be described through reference to FIGS. 10 and 11.

As shown in FIG. 10, when an element deactivation signal E3 with a rectangular waveform is transmitted from a controller to a deactivation switch, noise (electromagnetic waves and so forth) can sometimes occur due to the leading edge and trailing edge of the element deactivation signal E3 with a rectangular waveform. In this case, the reception circuit acquires unnecessary acoustic wave signals G4 and G5 that are attributable to this noise.

Meanwhile, as shown in FIG. 11, in the third embodiment, when the waveform E2 of the element deactivation signal having a blunt or gradual shape is transmitted from the controller 31 a to the deactivation switch 39b, since the time period of the leading edge and trailing edge have the time constants $\tau6$ and $\tau7$ and are longer than with the element deactivation signal E3 with a rectangular waveform, noise (electromagnetic waves and so forth) attributable to a change in waveform is smaller (to the point that it can be ignored) by an amount proportional to the longer time period of the leading edge and trailing edge. In this case, the reception circuit 33 does not acquire the unnecessary acoustic wave signals G4 and G5 that are attributable to noise.

The following effects are obtained with the third embodiment.

In the third embodiment, as discussed above, the controller 31 a is configured so that the leading edge or trailing edge of the waveform E2 of the element deactivation signal for controlling the deactivation switch 39b has a shape that is blunter or more gradual than a rectangular waveform (the element deactivation signal E3). Consequently, this reduces the generation of noise (electromagnetic waves and so forth) attributable to controlling (driving) the deactivation switch 39b. As a result, even when the deactivation switch 39b is provided to make the potential substantially the same at both ends of the ultrasonic transducer 24, malfunctioning (vibration) of the ultrasonic transducer 24 can be effectively suppressed. The rest of the effects of the photoacoustic imaging device 102 in the third embodiment are the same as those of the photoacoustic imaging device 100 in the first embodiment.

In the illustrated embodiment, the photoacoustic imaging device 102 comprises the light source 21 that emits pulsed light at the subject 10, the ultrasonic transducer 24 that converts the vibration of the detection object 10a of the subject 10 that is generated according to the pulsed light to the electric signal, and the controller 31 a that selectively activates or deactivates the ultrasonic transducer 24, the controller 31 a deactivating the ultrasonic transducer 24 while the light source 21 emits the pulsed light.

The photoacoustic imaging device 102 can further comprise the reception circuit 33 that acquires the electrical signal from the ultrasonic transducer 24.

Also, with the photoacoustic imaging device 102, the controller 31 a can deactivate the ultrasonic transducer 24 before the light source 21 starts emitting the pulsed light, and activate the ultrasonic transducer 24 after the light source 21 stops emitting the pulsed light.

With the photoacoustic imaging device 102, the controller 31 a can activate the ultrasonic transducer 24 before the vibration of the detection object 10a of the subject 10 is transmitted to the ultrasonic transducer 24.

With the photoacoustic imaging device 102, the controller 31 a can deactivate the ultrasonic transducer 24 by setting potential at both ends 24a and 24b of the ultrasonic transducer 24 to be equal to each other.

The photoacoustic imaging device 102 can further comprise the deactivation switch 39b electrically connected to the ultrasonic transducer 24. The controller 31 a can set the potential at the both ends 24a and 24b of the ultrasonic transducer 24 to be equal to each other by the deactivation switch 39b.

With the photoacoustic imaging device 102, the controller 31 a can operate the deactivation switch 39b with the element deactivation signal E2 (e.g., control signal) having the leading edge (at time t11, t12) and the trailing edge (at time t15, t16).

With the photoacoustic imaging device 102, the leading edge of the control signal includes a gradual leading edge (at time t11, t12) and/or the trailing edge of the control signal includes a gradual trailing edge (at time t15, t16).

With the photoacoustic imaging device 102, the deactivation switch 39b can deactivate the ultrasonic transducer 24 in response to a level of the leading edge of the element deactivation signal E2 (e.g., the control signal) reaching a predetermined deactivation level. For example, when the element deactivation signal E2 has a wave shape as illustrated in FIG. 9, the predetermined deactivation level can be equal to or less than the amplitude (maximum amplitude) of the element deactivation signal E2.

With the photoacoustic imaging device 102, the deactivation switch 39b can activate the ultrasonic transducer 24 in response to a level of the trailing edge of the element deactivation signal E2 (e.g., the control signal) reaching a predetermined activation level. For example, when the element deactivation E2 has a wave shape as illustrated in FIG. 9, the predetermined activation level can be equal to or less than the amplitude (maximum amplitude) of the element deactivation E2.

With the photoacoustic imaging device 102, a level of the leading edge of the element deactivation signal E2 (e.g., the control signal) reaches a predetermined deactivation level (at time t12) to deactivate the ultrasonic transducer 24 before the light source 21 starts emitting the pulsed light (at time t13). For example, when the element deactivation signal E2 has a wave shape as illustrated in FIG. 9, the predetermined deactivation level can be equal to or less than the amplitude (maximum amplitude) of the element deactivation signal E2.

With the photoacoustic imaging device 102, a level of the trailing edge of the element deactivation signal E2 (e.g., the control signal) reaching a predetermined activation level (at t16) to activate the ultrasonic transducer 24 after the light source 21 stops emitting the pulsed light (at time t14). For example, when the element deactivation E2 has a wave shape as illustrated in FIG. 9, the predetermined activation level can be equal to or less than the amplitude (maximum amplitude) of the element deactivation E2.

The photoacoustic imaging device 102 can further comprise the probe 20b housing the light source 21, the ultrasonic transducer 24 and the deactivation switch 39b inside of the probe 20b.

With the photoacoustic imaging device 102, the light source 21 can include the light emitting diode elements 21 a and 21 b that emits the pulsed light.

The embodiments disclosed herein are all just examples, and should not be construed as limiting in nature. The scope of the invention being indicated by the appended claims rather than by the above description of the embodiments, all modifications within the meaning and range of equivalency of the claims are included.

For example, in the first to third embodiments above, a light emitting diode element is used as the light emitting element of the present invention, but the present invention is not limited to this. For example, some light emitting element other than a light emitting diode element can be used as the light emitting element. In particular, a laser diode element can be used as the light emitting element.

Also, in the first to third embodiments above, pulsed light with a wavelength in the near infrared band is used as an example of the pulsed light that irradiates the subject of the present invention, but the present invention is not limited to this. For example, pulsed light with a wavelength outside of the near infrared band can be used as the pulsed light that irradiates the subject.

Also, in the first to third embodiments above, pulsed light with a pulse width of at least 100 ns and less than 200 ns is used as an example of the pulsed light that irradiates the subject of the present invention, but the present invention is not limited to this. For example, pulsed light with a pulse width outside the range of at least 100 ns and less than 200 ns can be used as the pulsed light that irradiates the subject. In particular, the pulsed light that irradiates the subject can be pulsed light with a pulse width of less than 100 ns, or can be pulsed light with a pulse width of 200 ns or more.

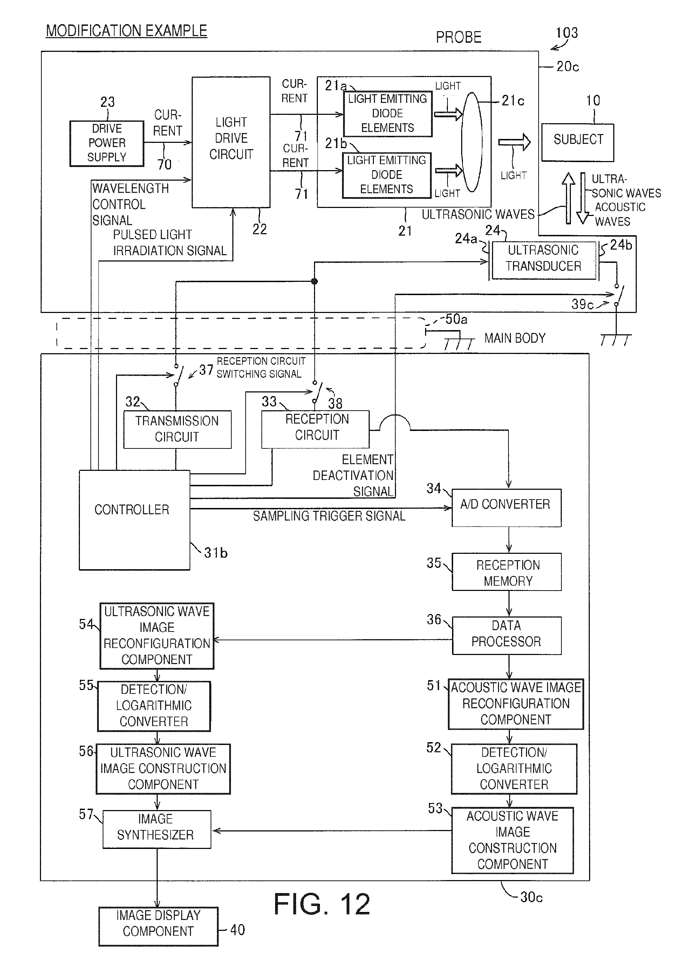

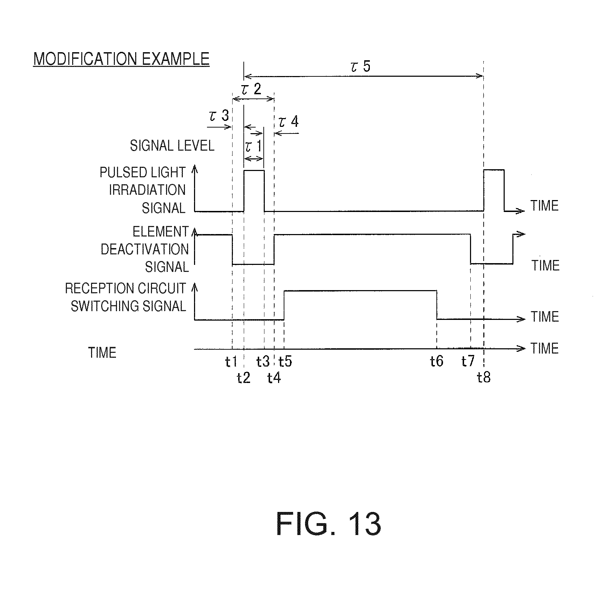

Also, in the first to third embodiments above, a deactivation switch that is connected at one end to one end of an ultrasonic transducer and is grounded at the other end is provided as an example of making the potential substantially the same at both ends of the ultrasonic transducer, and this deactivation switch is switched on (closed) to deactivate the ultrasonic transducer, but the present invention is not limited to this. For example, the ultrasonic transducer can be deactivated by some method other than grounding both ends of the ultrasonic transducer. For instance, as in the modification example shown in FIGS. 12 and 13, the configuration can be such that a deactivation switch 39c is provided between ground (earth) and the second end 24b of the ultrasonic transducer 24, and the deactivation switch 39c is switched off (opened) for a specific time period (the time period τ2 in the first embodiment) to deactivate the ultrasonic transducer 24.

As shown in FIG. 12, the photoacoustic imaging device 103 in accordance with a modification example includes a probe 20c and a main body 30c that is connected via a cable 50a to the probe 20c. The probe 20c includes a deactivation switch 39c. The main body 30c also includes controller 31 b. One side of the deactivation switch 39c is connected to the second end 24b of the ultrasonic transducer 24, and the other side of the deactivation switch 39c is grounded. As shown in FIG. 13, the controller 31 c makes the potential substantially the same at the first end 24a and the second end 24b of the ultrasonic transducer 24 by switching off (opening) the deactivation switch 39c for a specific time period (the time period τ2 in the first embodiment).

In the illustrated embodiment, the photoacoustic imaging device 103 comprises the light source 21 that emits pulsed light at the subject 10, the ultrasonic transducer 24 that converts the vibration of the detection object 10a of the subject 10 that is generated according to the pulsed light to the electric signal, and the controller 31 b that selectively activates or deactivates the ultrasonic transducer 24, the controller 31 b deactivating the ultrasonic transducer 24 while the light source 21 emits the pulsed light.

The photoacoustic imaging device 103 can further comprise the reception circuit 33 that acquires the electrical signal from the ultrasonic transducer 24.

Also, with the photoacoustic imaging device 103, the controller 31 b can deactivate the ultrasonic transducer 24 before the light source 21 starts emitting the pulsed light, and activate the ultrasonic transducer 24 after the light source 21 stops emitting the pulsed light.

With the photoacoustic imaging device 103, the controller 31 b can activate the ultrasonic transducer 24 before the vibration of the detection object 10a of the subject 10 is transmitted to the ultrasonic transducer 24.

With the photoacoustic imaging device 103, the controller 31 b can deactivate the ultrasonic transducer 24 by setting potential at both ends 24a and 24b of the ultrasonic transducer 24 to be equal to each other.

The photoacoustic imaging device 103 can further comprise the deactivation switch 39c electrically connected to the ultrasonic transducer 24. The controller 31 b can set the potential at the both ends 24a and 24b of the ultrasonic transducer 24 to be equal to each other by the deactivation switch 39c.

With the photoacoustic imaging device 103, the controller 31 b can operate the deactivation switch 39c with the element deactivation signal (e.g., control signal) having the leading edge (at time t1) and the trailing edge (at time t4).

With the photoacoustic imaging device 103, the deactivation switch 39c can deactivate the ultrasonic transducer 24 in response to a level of the leading edge of the element deactivation signal (e.g., the control signal) reaching a predetermined deactivation level. For example, as illustrated in FIG. 13, when the element deactivation signal has a rectangular wave with a low level and a high level, the predetermined deactivation level can be equal to or slightly more than the low level of the rectangular wave.

With the photoacoustic imaging device 103, the deactivation switch 39c can activate the ultrasonic transducer 24 in response to a level of the trailing edge of the element deactivation signal (e.g., the control signal) reaching a predetermined activation level. For example, as illustrated in FIG. 13, when the element deactivation signal has a rectangular wave with a low level and a high level, the predetermined activation level can be equal to or slightly less than the high level of the rectangular wave.

With the photoacoustic imaging device 103, a level of the leading edge of the element deactivation signal (e.g., the control signal) reaches a predetermined deactivation level (at time t1) to deactivate the ultrasonic transducer 24 before the light source 21 starts emitting the pulsed light (at time t2). For example, as illustrated in FIG. 13, when the element deactivation signal has a rectangular wave with a low level and a high level, the predetermined deactivation level can be equal to or slightly more than the low level of the rectangular wave.

With the photoacoustic imaging device 103, a level of the trailing edge of the element deactivation signal (e.g., the control signal) reaches a predetermined activation level (at time t4) to activate the ultrasonic transducer 24 after the light source 21 stops emitting the pulsed light (at time t3). For example, as illustrated in FIG. 13, when the element deactivation signal has a rectangular wave with a low level and a high level, the predetermined activation level can be equal to or slightly less than the high level of the rectangular wave.

The photoacoustic imaging device 103 can further comprise the probe 20c housing the light source 21, the ultrasonic transducer 24 and the deactivation switch 39c inside of the probe 20c.

With the photoacoustic imaging device 103, the light source 21 can include the light emitting diode elements 21 a and 21 b that emits the pulsed light.

Also, in the first to third embodiments above, the level of the element deactivation signal can be gradually increased (decreased) to increase the time constant of the leading edge and trailing edge and create a shape that is blunter or more gradual than a rectangular waveform, as an example of a blunt or gradual shape of the leading edge and trailing edge of the waveform of the element deactivation signal in the present invention, but the present invention is not limited to this. For example, the time constant of the leading edge and trailing edge can be increased to create a shape that is blunter or more gradual than a rectangular waveform by some method other than gradually increasing (decreasing) the level of the element deactivation signal. For instance, the time constant of the leading edge (trailing edge) can be substantially increased by gradually increasing (decreasing) the duty of the element deactivation signal and thereby gradually increasing (decreasing) the amount of current that can flow through the deactivation switch.

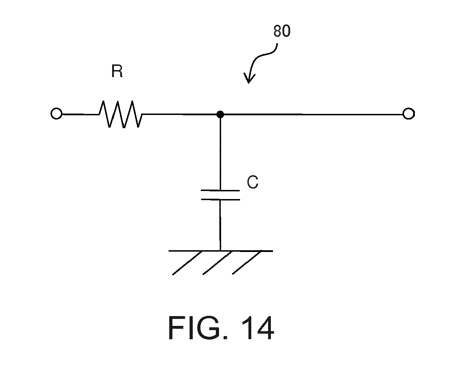

Also, in the first to third embodiments above, the generation of noise attributable to the deactivation switch is suppressed by a configuration in which the leading edge and trailing edge of the waveform of the element deactivation signal in the present invention have a shape that is blunter or more gradual than a rectangular waveform, but the present invention is not limited to this. For example, the configuration can be such that the leading edge and trailing edge of something other than the waveform of the element deactivation signal has a shape that is blunter or more gradual than a rectangular waveform. For instance, the generation of noise attributable to the operation of the deactivation switch can be suppressed by providing a circuit that includes a resistor and a capacitor, an inductor, or the like, in between the ultrasonic transducer and the deactivation switch, and thereby blunting the waveform of the current that flows between the ultrasonic transducer and the deactivation switch. In particular, FIG. 14 is a diagram illustrating the configuration of a low-pass filter 80 for the element deactivation signal. The low-pass filter 80 can be electrically connected between the controller 31 and the deactivation switch 39 in the photoacoustic imaging device 100 shown in FIG. 1, between the controller 31 and the deactivation switch 39a in the photoacoustic imaging device 101 shown in FIG. 8, between the controller 31 a and the deactivation switch 39b in the photoacoustic imaging device 102 shown in FIG. 8, and between the controller 31 b and the deactivation switch 39c in the photoacoustic imaging device 103 shown in FIG. 12. With this arrangements, the element deactivation signal for controlling the deactivation switch (39, 39a, 39b, 39c) from the controller (31, 31 a, 31 b) have a shape that is blunter or more gradual than a rectangular waveform, as shown in FIG. 9 and 11, after flowing through the low-pass filter 80. In the illustrated embodiment, the low-pass filter 80 is a first order RC filter, as illustrated in FIG. 14. As shown in FIG. 14, the low-pass filter 80 has a resistor R in series with a load, and a capacitor C in parallel with the load. Specifically, the resistor R has one end that is connected to the controller (31, 31 a, 31 b) and the other end that is connected to the capacitor C and the deactivation switch (39, 39a, 39b, 39c), while the capacitor C has one end that is connected to the other end of the resistor R and the deactivation switch (39, 39a, 39b, 39c) and the other end that is grounded. The low-pass filter 80 can be electrically disposed on either the main body (30, 30a, 30b, 30c) or the probe (20, 20a, 20b, 20c). In the illustrated embodiment, the first order RC filter (low-pass filter 80) has the time constant $\tau$ = C*R. The ultrasonic transducer 24 can be configured such that the time constant $\tau6$ and the time constant $\tau7$ (see FIGS. 9 and 11) are equal to C*R. In other words, the ultrasonic transducer 24 is deactivated when the deactivation switch (39, 39a, 39b) is turned on (or the deactivation switch (39c) is turned off) in response to charging the capacitor C to 63.2 percent of full charge (e.g., a predetermined deactivation level) (the time t12 in FIG. 9), while the ultrasonic transducer 24 is activated when the deactivation switch (39, 39a, 39b) is turned off (or the deactivation switch (39c) is turned on) in response to discharging the capacitor C to 36.8 percent of full charge (e.g., a predetermined activation level) (the time t16 in FIG. 9). Of course, the low-pass filter 80 can be any types of conventional low-pass filter, such as LC filter, RLC filter and the like. Also, the low-pass filter 80 can be an active low-pass filter using an operational amplifier.

Also, in the first to third embodiments above, a field effect transistor is used as the deactivation switch, but the present invention is not limited to this. For example, some switch other than a field effect transistor can be used. For instance, a bipolar transistor can be used as the deactivation switch.

In the illustrated embodiments, the photoacoustic imaging device in accordance with one aspect comprises a light source that includes a light emitting element capable of generating pulsed light, and that is able to emit the pulsed light at a subject, an ultrasonic transducer that vibrates under acoustic waves generated from a detection object inside the subject according to the pulsed light that is emitted, and that produces an acoustic wave signal corresponding to the vibration of the acoustic waves, a reception circuit that acquires the acoustic wave signal from the ultrasonic transducer, and a controller configured so as to perform control that deactivates the ultrasonic transducer for a specific time period that overlaps the time period in which the light emitting element generates the pulsed light.

With the photoacoustic imaging device, as mentioned above, the controller is configured to perform control that deactivates the ultrasonic transducer for a specific time period that overlaps the time period in which the light emitting element generates the pulsed light. Consequently, the ultrasonic transducer is deactivated for a specific time period that overlaps the time period in which noise (electromagnetic waves and so forth) attributable to the flow of current for generating pulsed light to the light emitting element is produced. Thus, the ultrasonic transducer will be less likely to malfunction (vibrate) on account of noise. As a result, even when the light source is disposed near the subject, the ultrasonic transducer and the reception circuit will be less likely to acquire a signal that has been affected by noise.

With the photoacoustic imaging device, it is preferable if the specific time period includes at least the time period in which the light emitting element generates the pulsed light. With this configuration, since the ultrasonic transducer is deactivated during the time period in which the pulsed light is generated, it is even less likely that the ultrasonic transducer will malfunction (vibrate).

In this case, it is preferable if the specific time period includes a time period before and after the time period in which the light emitting element generates the pulsed light, in addition to the time period in which the light emitting element generates the pulsed light. With this configuration, since the ultrasonic transducer is deactivated not only during the time period in which the pulsed light is generated, but also during a time period before and after the time period in which the light emitting element generates the pulsed light, the ultrasonic transducer is more effectively prevented from malfunctioning (vibrating).

With the photoacoustic imaging device configured so that the above-mentioned specific time period includes a time period in which the light emitting element generates the pulsed light, it is preferable if the controller is configured to perform control so that the reception circuit starts acquiring the acoustic wave signal after the time period in which the light emitting element generates the pulsed light, and the specific time period includes the time period in which the light emitting element generates the pulsed light, and also consists of a time period prior to when the reception circuit starts acquiring the acoustic wave signal. With this configuration, since the specific time period ends by the time the reception circuit starts acquiring an acoustic wave signal, it is less likely that the time period in which the reception circuit acquires the acoustic wave signal will be reduced by providing the specific time period in which the ultrasonic transducer is deactivated.

With the photoacoustic imaging device, it is preferable if the controller is configured to perform control so as to deactivate the ultrasonic transducer by setting the potential to be substantially the same at both ends of the ultrasonic transducer for the specific time period. With this configuration, since there is no potential difference between the ends of the ultrasonic transducer, the ultrasonic transducer can be easily deactivated.

In this case, it is preferable if there is further provided a switching component that is connected to the ultrasonic transducer, wherein the controller is configured to perform control so as to set the potential to be substantially the same at both ends of the ultrasonic transducer by controlling the switching component. With this configuration, the potential can be easily made substantially the same at both ends of the ultrasonic transducer by controlling the switching component.

With the photoacoustic imaging device, it is preferable if the controller is configured so that the leading edge and/or trailing edge of the waveform of a control signal for controlling the switching component has a shape that is blunter than a rectangular waveform. With this configuration, it is less likely that noise (electromagnetic waves and so forth) will be generated due to control (drive) of the switching component. As a result, even when a switching component is provided to make the potential substantially the same at both ends of the ultrasonic transducer, malfunction (vibration) of the ultrasonic transducer can be effectively suppressed.

With the photoacoustic imaging device comprising a switching component, it is preferable if there is further provided a probe that is configured to be able to emit the pulsed light from the light source at the subject by having the light source and the ultrasonic transducer disposed in the interior, and disposed near the subject, wherein the switching component is disposed in the interior of the probe. With this configuration, since the ultrasonic transducer and the switching component can be disposed close together, impedance (resistance, floating capacity, etc.) between the ultrasonic transducer and the switching component can be made lower than when the ultrasonic transducer and the switching component are disposed farther apart. As a result, the potential at both ends of the ultrasonic transducer can more reliably be set to be substantially the same.

With the photoacoustic imaging device comprising a switching component, it is preferable if there is further provided a probe that is configured to be able to emit the pulsed light from the light source at the subject by having the light source and the ultrasonic transducer disposed in the interior, and disposed near the subject, and a device main body that is connected via a cable to the probe, and in the interior of which are disposed the reception circuit and the controller, wherein the switching component is disposed in the interior of the device main body. With this configuration, unlike when the switching component is disposed on the probe, there is no need to install a cable to control the switching component. As a result, to the extent that no cable is needed for controlling the switching component, the configuration of the photoacoustic imaging device can be correspondingly simplified.

With the photoacoustic imaging device, it is preferable if the light emitting element is constituted by a light emitting diode element capable of generating the pulsed light. With this configuration, unlike when using a light emitting element that emits a laser beam, there is no need for precise alignment (positioning) of the optical members, nor are an optical bench and a sturdy housing required for suppressing fluctuation of the characteristics due to vibration of the optical system. As a result, since there is no need for precise alignment of optical members, and no need for an optical bench or a sturdy housing, the size and complexity of the photoacoustic imaging device can be correspondingly reduced.

With the present invention, as discussed above, it is less likely that a signal that has been affected by noise will be acquired by the ultrasonic transducer and the reception circuit, even when the light source is disposed near the subject.

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts unless otherwise stated.

While only selected embodiments have been chosen to illustrate the present invention, it will be apparent to those skilled in the art from this disclosure that various changes and modifications can be made herein without departing from the scope of the invention as defined in the appended claims. For example, unless specifically stated otherwise, the size, shape, location or orientation of the various components can be changed as needed and/or desired so long as the changes do not substantially affect their intended function. Unless specifically stated otherwise, components that are shown directly connected or contacting each other can have intermediate structures disposed between them so long as the changes do not substantially affect their intended function. The functions of one element can be performed by two, and vice versa unless specifically stated otherwise. The structures and functions of one embodiment can be adopted in another embodiment. It is not necessary for all advantages to be present in a particular embodiment at the same time. Every feature which is unique from the prior art, alone or in combination with other features, also should be considered a separate description of further inventions by the applicant, including the structural and/or functional concepts embodied by such feature(s). Thus, the foregoing descriptions of the embodiments according to the present invention are provided for illustration only, and not for the purpose of limiting the invention as defined by the appended claims and their equivalents.

**Claims**

1. A photoacoustic imaging device (100) comprising:

   a light source (21) configured to emit pulsed light at a subject; and
   an ultrasonic transducer (24) configured to convert vibration of a detection object of the subject that is generated according to the pulsed light to an electric signal;

**characterised by**

a controller (31) configured to selectively activate or deactivate the ultrasonic transducer (24), the controller (31) deactivating the ultrasonic transducer (24) while the light source (21) emits the pulsed light.

2. The photoacoustic imaging device (100) according to claim 1, further comprising a reception circuit (33) configured to acquire the electrical signal from the ultrasonic transducer (24).

3. The photoacoustic imaging device (100) according to claim 1 or 2, wherein the controller (31) is configured to deactivate the ultrasonic transducer (24) before the light source (21) starts emitting the pulsed light, and activate the ultrasonic transducer (24) after the light source (21) stops emitting the pulsed light.

4. The photoacoustic imaging device (100) according to any one of claims 1 to 3, wherein the controller (31) is configured to activate the ultrasonic transducer (24) before the vibration of the detection object of the subject is transmitted to the ultrasonic transducer (24).

5. The photoacoustic imaging device (100) according to any one of claims 1 to 4, wherein the controller (31) deactivates the ultrasonic transducer (24) by setting potential at both ends of the ultrasonic transducer (24) to be equal to each other.

6. The photoacoustic imaging device (100) according to any one of claims 1 to 5, further comprising a switch (39) electrically connected to the ultrasonic transducer (24), the controller (31) is configured to set the potential at the both ends of the ultrasonic transducer (24) to be equal to each other by the switch.

7. The photoacoustic imaging device (100) according to claim 6, further comprising a low-pass filter (80) electrically connected between the controller (31) and the switch (39).

8. The photoacoustic imaging device (100) according to claim 6 or 7, wherein the controller (31) is configured to operate the switch (39) with a control signal having a leading edge and a trailing edge.

9. The photoacoustic imaging device (100) according to any one of claims 6 to 8, wherein the controller (31) is configured to operate the switch (39) such that the leading edge of the control signal includes a gradual leading edge and/or the trailing edge of the control signal includes a gradual trailing edge.

10. The photoacoustic imaging device (100) according to claim 8 or 9, wherein the controller (31) is configured to operate the switch (39) such that the switch (39) deactivates the ultrasonic transducer (24) in response to a level of the leading edge of the control signal reaching a predetermined deactivation level.

11. The photoacoustic imaging device (100) according to any one of claims 8 to 10, wherein the controller (31) is configured to operate the switch (39) such that the switch (39) activates the ultrasonic transducer (24) in response to a level of the trailing edge of the control signal reaching a predetermined activation level.

12. The photoacoustic imaging device (100) according to any one of claims 8 to 11, wherein the controller (31) is configured to operate the switch (39) such that a level of the leading edge of the control signal reaches a predetermined deactivation level to deactivate the ultrasonic transducer (24) before the light source (21) starts emitting the pulsed light.

13. The photoacoustic imaging device (100) according to any one of claims 8 to 12, wherein the controller (31) is configured to operate the switch (39) such that a level of the trailing edge of the control signal reaching a predetermined activation level to activate the ultrasonic transducer (24) after the light source (21) stops emitting the pulsed light.

14. The photoacoustic imaging device (100) according to any one of claims 6 to 13, further comprising a probe (20) housing the light source (21), the ultrasonic transducer (24) and the switch (39) inside of the probe.

15. The photoacoustic imaging device (100) according to any one of claims 6 to 13, further comprising a probe (20) housing the light source (21) and the ultrasonic transducer (24) inside of the probe (20); and a device main body (30) connected via a cable to the probe (20), with the controller (31) and the switch (38) being disposed inside of the device main body (30).

FIG. 1

FIG. 2

FIRST EMBODIMENT

FIG. 3

COMPARATIVE PHOTOACOUSTIC IMAGING DEVICE

FIG. 4

COMPARATIVE PHOTOACOUSTIC
IMAGING DEVICE

FIG. 5

FIG. 6

FIG. 7

SECOND EMBODIMENT(THIRD EMBODIMENT)

FIG. 8

THIRD EMBODIMENT

FIG. 9

FIG. 10

THIRD EMBODIMENT

FIG. 11

MODIFICATION EXAMPLE PROBE 103

20c

23
DRIVE POWER SUPPLY

CUR-RENT
70

22
LIGHT DRIVE CIRCUIT

CUR-RENT

71 CUR-RENT

71

21a
LIGHT EMITTING DIODE ELEMENTS

LIGHT

21b
LIGHT EMITTING DIODE ELEMENTS

LIGHT

21c

21

LIGHT

ULTRASONIC WAVES

10
SUBJECT

LIGHT

ULTRA-SONIC WAVES ACOUSTIC WAVES

WAVELENGTH CONTROL SIGNAL
PULSED LIGHT IRRADIATION SIGNAL

24a 24
24b
ULTRASONIC TRANSDUCER

39c

50a
MAIN BODY

37 RECEPTION CIRCUIT SWITCHING SIGNAL

32
TRANSMISSION CIRCUIT

33 38
RECEPTION CIRCUIT

ELEMENT DEACTIVATION SIGNAL

CONTROLLER

SAMPLING TRIGGER SIGNAL

31b

34
A/D CONVERTER

35
RECEPTION MEMORY

36
DATA PROCESSOR

54
ULTRASONIC WAVE IMAGE RECONFIGURATION COMPONENT

55
DETECTION/ LOGARITHMIC CONVERTER

56
ULTRASONIC WAVE IMAGE CONSTRUCTION COMPONENT

57
IMAGE SYNTHESIZER

51
ACOUSTIC WAVE IMAGE RECONFIGURATION COMPONENT

52
DETECTION/ LOGARITHMIC CONVERTER

53
ACOUSTIC WAVE IMAGE CONSTRUCTION COMPONENT

30c

FIG. 12

IMAGE DISPLAY COMPONENT    40

MODIFICATION EXAMPLE

FIG. 13

FIG. 14

Europäisches
Patentamt

European
Patent Office

Office européen
des brevets

# EUROPEAN SEARCH REPORT

Application Number

EP 15 16 3580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2013 051403 A (FUJIFILM CORP) 14 March 2013 (2013-03-14) * abstract; figures 1,2,7,9,10 * * paragraphs [0026] - [0043], [0052] - [0062], [0072] - [0089] * | 1-15 | INV. G01N29/24 A61B5/00 G01N21/17 |
| X | XUEDING WANG ET AL: "A high-speed photoacoustic tomography system based on a commercial ultrasound and a custom transducer array", PROCEEDINGS OF SPIE, vol. 7564, 11 February 2010 (2010-02-11), pages 756424-756424-9, XP055164311, ISSN: 0277-786X, DOI: 10.1117/12.842666 * abstract; figure 1 * * page 2, left-hand column * | 1 | |
| X | CHRISTOPH HAISCH ET AL: "Combined optoacoustic/ultrasound system for tomographic absorption measurements: possibilities and limitations", ANALYTICAL AND BIOANALYTICAL CHEMISTRY, SPRINGER, BERLIN, DE, vol. 397, no. 4, 12 April 2010 (2010-04-12), pages 1503-1510, XP019839223, ISSN: 1618-2650 * abstract; figure 1 * * page 1505, left-hand column, last paragraph - right-hand column, paragraph 1 * | 1 | TECHNICAL FIELDS SEARCHED (IPC) G01N A61B G01S |

The present search report has been drawn up for all claims

| Place of search | Date of completion of the search | Examiner |
|---|---|---|
| Munich | 4 September 2015 | Uttenthaler, Erich |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                EP 15 16 3580

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2015

| Patent document cited in search report | Publication date | Patent family member(s) | | Publication date |
|---|---|---|---|---|
| JP 2013051403 A | 14-03-2013 | CN | 103718395 A | 09-04-2014 |
| | | EP | 2744052 A1 | 18-06-2014 |
| | | JP | 5662973 B2 | 04-02-2015 |
| | | JP | 2013051403 A | 14-03-2013 |
| | | US | 2014148680 A1 | 29-05-2014 |
| | | WO | 2013018632 A1 | 07-02-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014086369 A
- JP 2010042158 A